(19) Europäisches Patentamt
European Patent Office
Office européen des brevets

(11) **EP 2 059 278 B1**

(12) **EUROPÄISCHE PATENTSCHRIFT**

(45) Veröffentlichungstag und Bekanntmachung des Hinweises auf die Patenterteilung:
**15.03.2017 Patentblatt 2017/11**

(21) Anmeldenummer: **07801856.1**

(22) Anmeldetag: **23.08.2007**

(51) Int Cl.:
*A61M 1/36* (2006.01)      *A61M 5/145* (2006.01)

(86) Internationale Anmeldenummer:
**PCT/EP2007/007428**

(87) Internationale Veröffentlichungsnummer:
**WO 2008/022792 (28.02.2008 Gazette 2008/09)**

(54) **VERFAHREN UND VORRICHTUNG ZUM BEFÜLLEN EINER ZUGABEVORRICHTUNG EINES THERAPIEGERÄTES**

METHOD AND DEVICE FOR FILLING A SUPPLY DEVICE OF A THERAPY APPLIANCE

PROCÉDÉ ET DISPOSITIF POUR REMPLIR UN DISPOSITIF D'ALIMENTATION D'UN APPAREIL THÉRAPEUTIQUE

(84) Benannte Vertragsstaaten:
**AT BE BG CH CY CZ DE DK EE ES FI FR GB GR HU IE IS IT LI LT LU LV MC MT NL PL PT RO SE SI SK TR**

(30) Priorität: **24.08.2006 DE 102006039675**

(43) Veröffentlichungstag der Anmeldung:
**20.05.2009 Patentblatt 2009/21**

(73) Patentinhaber: **Fresenius Medical Care Deutschland GmbH**
**61352 Bad Homburg (DE)**

(72) Erfinder: **KOPPERSCHMIDT, Pascal**
**97456 Dittelbrunn (DE)**

(74) Vertreter: **Herrmann, Uwe et al**
**Lorenz Seidler Gossel**
**Rechtsanwälte Patentanwälte**
**Partnerschaft mbB**
**Widenmayerstraße 23**
**80538 München (DE)**

(56) Entgegenhaltungen:
WO-A-96/09844          US-A- 5 116 316
US-A1- 2005 234 382

EP 2 059 278 B1

**Beschreibung**

Die vorliegende Erfindung betrifft ein Verfahren zum Befüllen einer Zugabevorrichtung eines Therapiegerätes, das einen extrakorporalen Kreislauf aufweist, mit dem die Zugabevorrichtung derart in Verbindung steht, dass mittels der Zugabevorrichtung während des Betriebes des Therapiegerätes ein Mittel in den extrakorporalen Kreislauf infundierbar ist, wobei das Verfahren den Schritt der Verdünnung eines in der Zugabevorrichtung befindlichen Konzentrats mit einem Verdünnungsmittel vor Beginn der Patientenbehandlung umfasst.

Bei extrakorporalen Blutbehandlungsverfahren werden beispielsweise Antikoagulationsmittel, insbesondere Heparin, eingesetzt, um eine Blutgerinnung während der Behandlung zu verhindern.

Die Zufuhr des Antikoagulationsmittels erfolgt üblicherweise über eine Spritzenpumpe, die das Antikoagulationsmittel unmittelbar in den extrakorporalen Blutkreislauf eines Therapiegerätes fördert.

Die in die Spritzenpumpe einzusetzenden Heparinspritzen, die üblicherweise ein Volumen von z. B. 30 ml aufweisen, werden nach dem Stand der Technik vom Dialysepersonal manuell zunächst mit einer kleinen Menge an konzentriertem Heparin befüllt, das dann durch manuelle Zugabe von physiologischer Kochsalzlösung auf die gewünschte Konzentration verdünnt wird.

Das Befüllen der Heparinpumpe erfolgt üblicherweise somit separat gemäß den Vorgaben des Arztes und patientenindividuell durch das Behandlungspersonal, bevor die gefüllte Spritze mit dem Blutschlauchsystem verbunden wird.

Das behandelnde Personal muss Menge und Konzentration des verdünnten Heparins in der Heparinspritze derart herstellen, dass mit einer bestimmten Förderrate der Spritzenpumpe eine bestimmte Dosis entsprechend der Medikation verabreicht werden kann. Die verschiedenen zu beachtenden Größen müssen durch das Personal manuell umgerechnet bzw. berechnet werden, wobei dieser Vorgang in der Regel für jeden Patienten individuell und separat vor Behandlungsbeginn entsprechend der ärztlich vorgegebenen Medikation erfolgt.

Für die Bereitstellung der mit verdünntem Heparin gefüllten Spritze werden für jeden Patienten Verbrauchsmittel benötigt, wie beispielsweise Einmalhandschuhe, eine Einwegspritze mit Nadel zur Umfüllung des konzentrierten Heparins sowie eine Flasche physiologischer Kochsalzlösung und Desinfektionsmittel. Der Vorgang dauert für jeden Patienten jeweils mehrere Minuten und ist nicht nur aufwendig, sondern wie ausgeführt, auch fehleranfällig.

Aus dem Stand der Technik ist es bekannt, dass mit einem Gemisch aus konzentriertem Antikoagulationsmittel bzw. Heparin und physiologischer Kochsalzlösung vorgefüllte Heparinspritzen an Dialysemaschinen in Spritzenpumpen eingelegt werden und während der Blutbehandlung entsprechend einer bestimmten Dosisvorgabe Heparin in den extrakorporalen Blutkreislauf, das heißt in das Patientenblut infundiert wird. Exemplarisch ist auf die U.S. 5,015,226 sowie auf die U.S. 2005/0234382 A1 zu verweisen.

Die US 5 116 316 offenbart ein Verfahren und eine Vorrichtung zum Befüllen einer Zugabevorrichtung eines Therapiegerätes.

J Es ist die Aufgabe der vorliegenden Erfindung ein Verfahren der eingangs genannten Art dahingehend weiterzubilden, dass sich die Vorbereitung der Zugabevorrichtung gegenüber aus dem Stand der Technik bekannten Vorgehensweisen einfacher gestaltet.

Diese Aufgabe wird durch ein Verfahren mit den Merkmalen des Anspruchs 1 gelöst. Danach ist vorgesehen, dass die Verdünnung des in der Zugabevorrichtung befindlichen Konzentrats dadurch erfolgt, dass die Zugabevorrichtung im an den extrakorporalen Kreislauf angeschlossenen Zustand aus dem extrakorporalen Kreislauf das Verdünnungsmittel aufnimmt. Aus dem Stand der Technik ist es unbekannt, beispielsweise eine Heparinspritze gezielt am extrakorporalen Blutkreislauf aufzuziehen bzw. am extrakorporalen Blutkreislauf mit einem Verdünnungsmittel beispielsweise physiologischer Kochsalzlösung zu befüllen. Im Stand der Technik bestand vielmehr das Vorurteil, dass sich eine Aufziehbewegung einer Spritze am extrakorporalen Blutkreislauf grundsätzlich verbietet.

Das erfindungsgemäße Verfahren weist unter anderem die Vorteile auf, dass sich die Befüllung der Zugabevorrichtung vergleichsweise einfach gestaltet, wodurch das Personal entlastet wird, das sich dementsprechend dann mehr auf den Patienten konzentrieren kann. Es kommt zu einer Zeitersparnis für das Personal, die sich letztlich in einer Kostenersparnis niederschlägt.

Im übrigen wird eine erhöhte Sicherheit gewährleistet, da das vergleichsweise umständliche Vorbereiten der Zugabevorrichtung, beispielsweise der Spritze, wie es aus dem Stand der Technik bekannt ist, entfällt.

Besonders vorteilhaft ist es, wenn die Zugabevorrichtung mit einer standardisierten Menge und/oder Konzentration des Konzentrats gefüllt ist. Denkbar ist somit, dass beispielsweise mit einer bestimmten, standardisierten Menge an konzentriertem Heparin vorgefüllte Spritzen in die Spritzenpumpe eingelegt werden. Vor Beginn der Behandlung wird durch Aufziehen von physiologischer Kochsalzlösung aus dem extrakorporalen Blutkreislauf das in der vorgefüllten Spritze befindliche Konzentrat auf den gewünschten Wert verdünnt. Erfolgt dieser Vorgang automatisiert, beispielsweise durch das Dialysegerät, wird der manuelle Arbeitsgang der Heparinverdünnung, wie er aus dem Stand der Technik bekannt ist, und vorzugsweise auch die dabei erforderliche Umrechnung durch das Behandlungspersonal überflüssig, wobei sich die Sicherheit für den Patienten erhöht und der Arbeitsaufwand für das Personal erheblich verringert.

In weiterer Ausgestaltung der Erfindung ist vorgesehen, dass es sich bei der Zugabevorrichtung um eine Spritzenpumpe mit einer darin eingesetzten Spritze handelt und dass das Aufnehmen des Verdünnungsmittels durch Aufziehen der Spritze erfolgt.

Denkbar ist es, die genannte standardisierte Menge und/oder Konzentration des Konzentrats werkseitig vorzusehen, so dass das Behandlungszentrum mit entsprechend vorgefüllten Spritzen versorgt wird. Grundsätzlich ist es ebenfalls denkbar, Spritzen chargenweise durch das Behandlungspersonal vorzubereiten und vor Ort zu bevorraten. Wie ausgeführt, ist auch eine Zulieferung standardmäßig mit Heparinpräparat vorgefüllter Spritzen durch einschlägige Spritzenhersteller denkbar. Selbstverständlich ist die Erfindung nicht auf die Verwendung von Heparin beschränkt. Jedes Konzentrat, das dem Patientenblut zuzuführen ist, ist im Rahmen der Erfindung einsetzbar.

Gemäß der Erfindung ist vorgesehen, dass das Aufnehmen des Verdünnungsmittels durch die Zugabevorrichtung während der Spülphase des extrakorporalen Kreislaufes und vorzugsweise kurz vor Beendigung der Spülphase des extrakorporalen Kreislaufes erfolgt.

In weiterer Ausgestaltung der Erfindung ist vorgesehen, dass das Spülen des extrakorporalen Kreislaufes mit Kochsalzlösung oder mit vorzugsweise online hergestelltem Dialysat erfolgt und dass es sich dementsprechend bei dem Verdünnungsmittel um Kochsalzlösung oder um Dialysat handelt.

Wie oben ausgeführt, handelt es sich bei dem Konzentrat vorzugsweise um ein Antikoagulationsmittel und besonders bevorzugt um Heparin.

In einer weiteren Ausgestaltung der Erfindung ist vorgesehen, dass ein mit dem extrakorporalen Kreislauf in Verbindung stehender Drucksensor angeordnet ist, der während der Aufnahme des Verdünnungsmittels durch die Zugabevorrichtung eine Überwachung des Druckes vornimmt, wobei bei einer Inkonsistenz zwischen dem durch die Zugabevorrichtung aufgenommenen Flüssigkeitsvolumen und dem gemessenen Druck eine Information des Anwenders erfolgt. Diese Information kann beispielsweise in Form eines Alarms bzw. einer akustischen oder optischen Anzeige erfolgen. Der Drucksensor kann somit verwendet werden, um Undichtigkeiten an der Konnektionsstelle zwischen dem extrakorporalen Blutkreislauf und einer Spritze bzw. einem von der Spritze zum extrakorporalen Blutkreislauf verlaufenden Schlauch drucküberwacht zu erkennen.

In einer weiteren Ausführungsform der Erfindung sind Eingabemittel vorgesehen, mittels derer die patientenindividuelle Konzentration des zuzugebenden Mittels eingebbar ist, wobei eine Steuereinheit vorgesehen ist, die automatisiert aufgrund der von den Eingabemitteln erhaltenen Daten das erforderliche Volumen des Verdünnungsmittels bestimmt und die Zugabevorrichtung derart ansteuert, dass diese das bestimmte Volumen des Verdünnungsmittels aufnimmt. Denkbar ist beispielsweise, dass eine patientenindividuelle Medikation von einer Karte oder einem sonstigen Speichermittel durch das Therapiegerät auslesbar ist bzw. an das Therapiegerät übertragen wird und auf der Grundlage dieser Informationen automatisiert das erforderliche Volumen des Verdünnungsmittels berechnet wird und dann mittels der Zugabevorrichtung aufgenommen wird.

Denkbar ist weiterhin, dass die Zugabevorrichtung mit einer von mehreren standardisierten Mengen und/oder Konzentrationen des Konzentrats gefüllt ist und dass das Therapiegerät Eingabemittel aufweist, in die eingebbar ist, um welches der standardisierten Konzentrate es sich handelt. Dem Therapiegerät ist lediglich die beispielsweise werkseitig oder seitens eines Behandlungszentrums festgelegte Heparinkonzentration mitzuteilen. Eine erneute Eingabe einer Konzentration ist nur bei Wechsel der Füllung der vorgefüllten Spritzen notwendig, nicht jedoch bei jeder Behandlung.

Es ist auch möglich, die Heparinspritze mit einem Erkennungsmerkmal auszustatten, so dass die Dialysemaschine die Eigenschaften einer Heparinspritze mit lieferantenseitiger Vorfüllung mittels bekannter optischer, mechanischer oder elektronischer Mittel automatisch erkennen kann.

Bei dem Therapiegerät handelt es sich vorzugsweise um ein Dialysegerät.

Die vorliegende Erfindung betrifft des weiteren eine Dialysemaschine nach Anspruch 11, die zur Aufnahme eines extrakorporalen Kreislaufes, beispielsweise eines Blutschlauchsatzes und/oder eines Kassettensystems geeignet ist. Das Therapiegerät weist eine Zugabevorrichtung für Konzentrat mit einem Verdünnungsmittel auf. Erfindungsgemäß ist vorgesehen, dass das Therapiegerät eine Steuereinheit und Eingabemittel aufweist bzw. mit diesen in Verbindung steht, wobei die Steuereinheit automatisiert aufgrund der von den Eingabemitteln erhaltenen Daten das erforderliche Volumen des Verdünnungsmittels bestimmt und die Zugabevorrichtung derart ansteuert, dass diese das bestimmte Volumen des Verdünnungsmittels aus dem extrakorporalen Blutkreislauf aufnimmt.

Weitere vorteilhafte Ausgestaltungen der Dialysemaschine sind Gegenstand der Unteransprüche 12 bis 21.

Weitere Einzelheiten und Vorteile der Erfindung werden anhand eines in der Zeichnung dargestellten Ausführungsbeispiels näher läutert. Es zeigen:

Figur 1:    eine schematische Darstellung einer mit dem extrakorporalen Blutkreislauf in Verbindung stehenden Spritzenpumpe und

Figur 2:    die zeitlichen Abläufe des Volumens und der Konzentration einer in einer Heparinspritze befindlichen Heparinlösung.

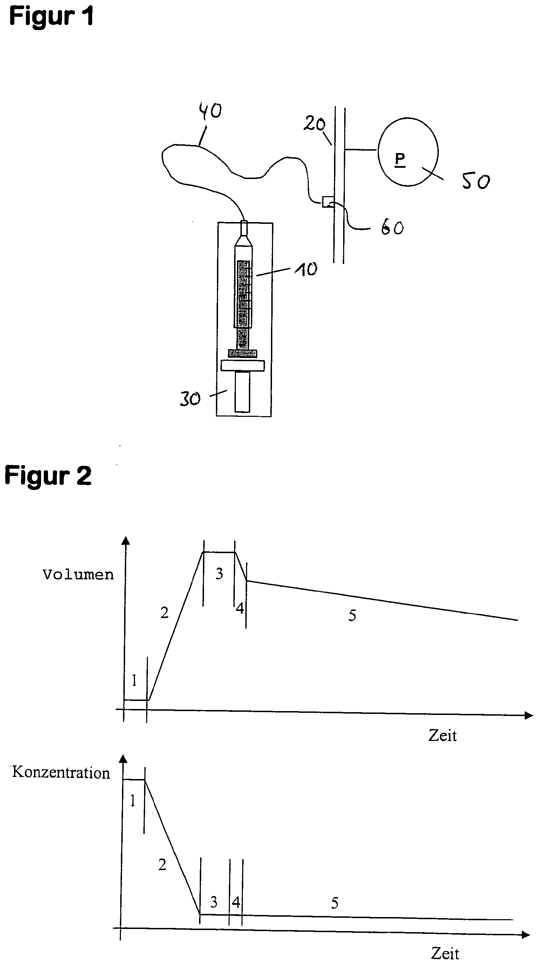

Gemäß einem bevorzugten Ausführungsbeispiel der vorliegenden Erfindung wird die aus Figur 1 ersichtliche Heparinspritze 10, die mit einer werkseitig festgelegten Heparinkonzentration vorgefüllt ist, während des Aufrüstens des Blutschlauchsystems 20 zur Durchführung der Therapieverfahrens/Dialyseverfahrens mit dem Schlauchsystem konnektiert und in die vorgesehene Fördereinheit, nämlich die Spritzenpumpe 30 eingelegt. Während der Spülphase des Blutschlauchsystems 20 mit physiologischer Flüssigkeit, vorzugsweise mit physiologischer Kochsalzlösung wird kurz vor Beendigung des Spülens die Heparinpumpe 30 mit der physiologischen Flüssigkeit gefüllt, bis eine definierte Heparinkonzentration in der Spritze 10 erreicht ist. Hierzu wird die werkseitig festgelegte Heparinkonzentration, die in der Spritze vorliegt, dem gemäß diesem Ausführungsbeispiel als Dialysegerät ausgeführten Therapiegerät mitgeteilt. Anhand der individuell verordneten Rezeptur für den Patienten berechnet das Therapiegerät sodann das erforderliche Volumen des Verdünnungsmittels und nimmt automatisiert eine entsprechende Betätigung der Spritze 10 dahingehend vor, dass diese über die Verbindungsleitung 40 aus dem extrakorporalen Blutkreislauf 20 das berechnete Volumen an physiologischer Flüssigkeit aufnimmt. In der Spritze 10 befindet sich sodann die vorgeschriebene Konzentration der verdünnten Heparinlösung.

Des weiteren kann vorgesehen sein, dass anhand einer individuell verordneten Rezeptur für den Patienten das Therapiegerät die Förderleistung der Heparinpumpe selbständig berechnet und gemäß einem definierten Profil dem zu therapierenden Blut während der Behandlung beimengt.

Im folgenden wird ein möglicher Verfahrensablauf zur Durchführung des erfindungsgemäßen Verfahrens vorgestellt:

> Die Heparindosis wird üblicherweise in Einheiten ‚Units' angegeben. Typische Gaben von Heparin bei Dialysebehandlungen liegen bei etwa 500 - 1.000 Units/Stunde. Einmalige Initialdosen liegen bei 1.000 - 3.000 Units. Heparin wird in Glasfläschchen von 5 ml mit 25.000 Units angeboten. Eine werksseitig vorgefüllte 30 ml große Heparinspritze könnte demnach mit einer gewählten Dosis von 10.000 Units vorgefüllt sein. Dies entspräche einem Volumen von 10.000/25.000 * 5 ml = 2 ml. Diese Heparinspritzen können werksseitig oder im Dialysezentrum in größerer Anzahl vorgefüllt werden.

Die mit 2 ml Heparin vorgefüllte 30 ml Heparinspritze 10 wird während des Vorbereitens mit dem extrakorporale Blutschlauchsystem 20 verbunden und vor Beendigung des Spülvorganges mit frischer physiologischer Spüllösung, z. B. Dialysat, automatisch gefüllt. Beispielsweise kann die Heparinspritze mit 18 ml Spüllösung gefüllt werden, so daß eine Heparinkonzentration von 20.000 Units/20 ml = 1.000 Units/ml entsteht. Die maschinenseitige Befüllung der 30 ml Spritze sollte das Volumen von 25 ml nicht überschreiten. Erfordert die Rezeptur eine Initialdosis von 2.500 Units zu Beginn der Behandlung und eine kontinuierliche Gabe von 500 Units/Stunde, wird die Heparinpumpe 30 unmittelbar nach Behandlungsbeginn einen Bolus von 2,5 ml dem Blut beimengen. Kontinuierlich wird die Heparinspritze eine Förderrate von 0,5 ml/h bis zur gewünschten Stoppzeit leisten.

Wie dies aus Figur 1 weiter hervorgeht, ist mit dem extrakorporalen Blutschlauchsystem ein Drucksensor 50 verbunden, bei dem es sich um den ohnehin in der arteriellen Blutschlauchleitung befindlichen Drucksensor handeln kann. Im Rahmen des erfindungsgemäßen Verfahrens dient der Sensor 50 zur Überwachung der Befüllung der Spritze 10. Während des Aufziehens der Spritze 10 zur Aufnahme des Verdünnungsmittels aus dem Blutschlauchsystem 20 wird der Druck mittels des Sensors 50 überwacht. Sofern eine Inkonsistenz zwischen dem Druckabfall und der Füllung der Spritze 10 festgestellt wird, kann dies dem Anwender entsprechend mitgeteilt werden. Auf diese Weise kann die Dichtigkeit geprüft werden und es lassen sich Undichtigkeiten der Konnektionsstelle 60 zwischen dem Verbindungsschlauch 40 und dem extrakorporalen Blutschlauchsystem 20 drucküberwacht erkennen. Die Befüllung kann entsprechend dem Spülvorgang einem Kochsalzbeutel oder einem online aufbereiteten Dialysat entnommen werden.

Anstelle des Blutschlauchsystems 20 kann die Heparinspritze auch an ein Kassettensystem angeschlossen sein, das in Figur 1 nicht dargestellt ist.

Der Begriff des extrakorporalen Kreislaufes umfaßt im Rahmen der vorliegenden Erfindung beispielsweise das Blutschlauchsystem, ein Kassettensystem etc. bzw. beliebige sonstige Bestandteile und Komponenten des extrakorporalen Kreislaufes.

Figur 2 zeigt den zeitlichen Verlauf des in der Spritze 10 befindlichen Volumens (obere Darstellung) sowie den zeitlichen Verlauf der Heparinkonzentration der in der Spritze 10 befindlichen Heparinlösung (untere Darstellung).

Die Zeitzonen bezeichnen im einzelnen die Aufrüstphase 1, die Befüllung der Spritze 2, Konnektierung des Patienten 3, Heparin-Bolusgabe 4 und kontinuierliche Heparinisierung während der Behandlung 5.

Gemäß der Rezeptur des Arztes wird die Initialdosis in ‚Units' Heparin, die kontinuierliche Gabe in ‚Units/h', das Profil und die Stoppzeit dem Therapiegerät per Eingabe oder Patientenkarte übermittelt. Die Eingabe der Konzentration in der werksseitig gefüllten Heparinspritze ist nur bei Wechsel der Füllung der vorgefüllten Spritzen notwendig, nicht jedoch vor jeder Behandlung.

Über einfache Beziehungen werden die nach der geräteseitigen Befüllung der Heparinspritze die Konzentration an Heparin bestimmt und gemäß den Vorgaben des Arztes das Initialvolumen und die Förderrate bestimmt.

Geräteseitig werden folgende Rechenschritte beispielhaft durchgeführt:

Konzentration der mit 25 ml gefüllten Spritze:

$$c_{Hep} = \frac{10.000\ Units}{25\ ml} = 400\ \frac{Units}{ml}$$

Initialvolumen bei initialer Gabe von 2.000 Units:

$$V_{initHep} = \frac{2.000\ Units}{c_{Hep}} = 5\ ml$$

Kontinuierliche Förderrate bei Gabe von 500 Units/Stunde:

$$Q_{Hep} = \frac{500\ Units}{h} \cdot \frac{1}{c_{Hep}} = 1{,}25\ \frac{ml}{h}$$

Gemäß dem hier exemplarisch vorgestellten Ausführungsbeispiel wird der Vorteil erreicht, das ein Umrechnung von patientenspezifischer rezeptierter Gabe von Heparin von den Einheiten ‚Units' in ‚ml/Stunde' Förderrate wegfällt. Die erforderlichen Rechenschritte können geräteseitig durchgeführt werden. Dadurch lassen sich Rechenfehler zuverlässig vermeiden. Die Förderrate der Heparinpumpe ist dann für das Personal irrelevant und das Augenmerk kann allein auf die rezeptierte Gabe in den Einheiten ‚Units' reduziert werden.

Wie dies oben ausgeführt wurde, ergibt sich aufgrund der Verwendung werkseitig oder im Dialysezentrum vorgefüllter Heparinspritzen der Vorteil, dass das Befüllen mit einer individualisierten, auf den Patienten abgestimmten Menge Heparin und die Verwendung von zusätzlichen Verbrauchsmaterialien wegfällt. Durch die Befüllung der Zugabevorrichtung bzw. Spritze aus dem extrakorporalen Blutkreislauf während der Vorbereitungsphase wird der Verfahrensablauf gegenüber aus dem Stand der Technik bekannten Lösungen wesentlich vereinfacht. Ein weiterer Vorteil ergibt sich daraus, dass gleichzeitig Konnektionsstellen und Zuleitungen auf Dichtigkeit und Durchlass geräteseitig geprüft werden können, wozu der oben genannte Drucksensor eingesetzt werden kann. Durch die standardisierte Vorbereitung von Heparinspritzen ergibt sich eine Ersparnis an Verbrauchsmaterial und damit ein Kostenvorteil. Vorzugsweise wird das erfindungsgemäße Verfahren automatisch durchgeführt. Das heißt ein Eingriff des Anwenders ist vorzugsweise nicht erforderlich.

## Patentansprüche

1. Verfahren zum Befüllen einer Zugabevorrichtung eines Therapiegerätes, das einen extrakorporalen Kreislauf (20) aufweist, mit dem die Zugabevorrichtung derart in Verbindung steht, dass mittels der Zugabevorrichtung während des Betriebes des Therapiegerätes ein Mittel in den extrakorporalen Kreislauf (20) einführbar ist, wobei das Verfahren den Schritt der Verdünnung eines in der Zugabevorrichtung befindlichen Konzentrats mit einem Verdünnungsmittel umfasst, wobei die Verdünnung des in der Zugabevorrichtung befindlichen Konzentrats dadurch erfolgt, dass die Zugabevorrichtung im an den extrakorporalen Kreislauf (20) angeschlossenen Zustand aus dem extrakorporalen Kreislauf (20) das Verdünnungsmittel aufnimmt, **dadurch gekennzeichnet, dass** das Verfahren den Schritt des Spülens des extrakorporalen Kreislaufs umfasst und dass der extrakorporale Kreislauf (20) einen Blutschlauchsatz umfasst oder aus diesem besteht und dass das Aufnehmen des Verdünnungsmittels durch die Zugabevorrichtung vor Beginn der Behandlung und während der Spülphase des extrakorporalen Kreislaufes (20) und vorzugsweise kurz vor Beendigung der Spülphase des extrakorporalen Kreislaufes (20) erfolgt.

2. Verfahren nach Anspruch 1, **dadurch gekennzeichnet, dass** die Zugabevorrichtung mit einer standardisierten Menge und/oder Konzentration des Konzentrats gefüllt ist.

3. Verfahren nach Anspruch 1 oder 2, **dadurch gekennzeichnet, dass** es sich bei der Zugabevorrichtung um eine Spritzenpumpe (30) mit einer darin eingesetzten Spritze (10) handelt und dass das Aufnehmen des Verdünnungsmittels durch Aufziehen der Spritze (10) erfolgt.

4. Verfahren nach Anspruch 1, **dadurch gekennzeichnet, dass** das Spülen des extrakorporalen Kreislaufes (20) mit Kochsalzlösung oder mit vorzugsweise online hergestelltem Dialysat erfolgt und dass es sich dementsprechend bei dem Verdünnungsmittel um Kochsalzlösung oder um Dialysat handelt.

5. Verfahren nach einem der vorhergehenden Ansprüche, **dadurch gekennzeichnet, dass** es sich bei dem Konzentrat um ein Antikoagulationsmittel handelt.

6. Verfahren nach Anspruch 5, **dadurch gekennzeichnet, dass** es sich bei dem Antikoagulationsmittel um Heparin handelt.

7. Verfahren nach einem der vorhergehenden Ansprüche, **dadurch gekennzeichnet, dass** ein mit dem extrakorporalen Kreislauf (20) in Verbindung stehender Drucksensor (50) vorgesehen ist, dass während der Aufnahme des Verdünnungsmittels durch die Zugabevorrichtung eine Überwachung des mittels des Drucksensors (50) gemessenen Drucks erfolgt und dass bei einer Inkonsistenz zwischen dem durch die Zugabevorrichtung aufgenommenen Flüssigkeitsvolumen und dem gemessenen Druck eine Information des Anwenders erfolgt.

8. Verfahren nach einem der vorhergehenden Ansprüche, **dadurch gekennzeichnet, dass** Eingabemittel vorgesehen sind, mittels derer die patientenindividuelle Konzentration des zuzugebenden Mittels eingebbar ist und dass eine Steuereinheit vorgesehen ist, die automatisiert aufgrund der von den Eingabemitteln erhaltenen Daten das erforderliche Volumen des Verdünnungsmittels bestimmt und die Zugabevorrichtung derart ansteuert, dass diese das bestimmte Volumen des Verdünnungsmittels aufnimmt.

9. Verfahren nach Anspruch 8, **dadurch gekennzeichnet, dass** die Eingabemittel eine Leseeinrichtung umfassen, mittels derer eine patientenindividuelle Medikation von einer Karte oder einem sonstigen Speichermittel auslesbar ist.

10. Verfahren nach einem der vorhergehenden Ansprüche, **dadurch gekennzeichnet, dass** die Zugabevorrichtung mit einer von mehreren standardisierten Mengen und/oder Konzentrationen des Konzentrats gefüllt ist und dass das Therapiegerät Eingabemittel aufweist, in die eingebbar ist, um welches der standardisierten Konzentrate es sich handelt.

11. Dialysemaschine, geeignet zur Aufnahme eines extrakorporalen Kreislaufes (20) mit einer Zugabevorrichtung für Konzentrat mit einem Verdünnungsmittel, wobei die Dialysemaschine eine Steuereinheit sowie Eingabemittel aufweist, **dadurch gekennzeichnet, dass** die Steuereinheit derart ausgeführt ist, dass sie automatisiert aufgrund der von den Eingabemitteln erhaltenen Daten das erforderliche Volumen des Verdünnungsmittels bestimmt und die Zugabevorrichtung derart ansteuert, dass diese das bestimmte Volumen des Verdünnungsmittels aus dem extrakorporalen Kreislauf (20) aufnimmt, wobei die Steuereinheit derart ausgeführt ist, dass das Aufnehmen des Verdünnungsmittels durch die Zugabevorrichtung während der Spülphase des extrakorporalen Kreislaufes (20) und vorzugsweise kurz vor Beendigung der Spülphase des extrakorporalen Kreislaufes (20) erfolgt.

12. Dialysemaschine nach Anspruch 11, **dadurch gekennzeichnet, dass** die Zugabevorrichtung mit einer standardisierten Menge und Konzentration des Konzentrats gefüllt ist.

13. Dialysemaschine nach Anspruch 11 oder 12, **dadurch gekennzeichnet, dass** es sich bei der Zugabevorrichtung um eine Spritzenpumpe (30) mit einer darin eingesetzten Spritze (10) handelt, wobei die Zugabevorrichtung derart angeordnet ist, dass das Aufnehmen des Verdünnungsmittels aus dem extrakorporalen Kreislauf (20) durch Aufziehen der Spritze (10) erfolgt.

14. Dialysemaschine nach Anspruch 11, **dadurch gekennzeichnet, dass** als Spülmittel zum Spülen des extrakorporalen Kreislaufes (20) eine Kochsalzlösung oder vorzugsweise online hergestelltes Dialysat vorgesehen ist und dass es sich dementsprechend bei dem Verdünnungsmittel um Kochsalzlösung oder um Dialysat handelt.

15. Dialysemaschine nach einem der Ansprüche 11 bis 14, **dadurch gekennzeichnet, dass** es sich bei dem Konzentrat um ein Antikoagulationsmittel handelt.

16. Dialysemaschine nach Anspruch 15, **dadurch gekennzeichnet, dass** es sich bei dem Antikoagulationsmittel um Heparin handelt.

17. Dialysemaschine nach einem der Ansprüche 11 bis 16, **dadurch gekennzeichnet, dass** ein mit dem extrakorporalen Kreislauf (20) in Verbindung stehender Drucksensor (50) vorgesehen ist, dass Überwachungsmittel vorgesehen sind, mittels derer während der Aufnahme des Verdünnungsmittels durch die Zugabevorrichtung eine Überwachung des Mittels des Drucksensors (50) gemessenen Drucks erfolgt, und dass Informationsmittel vorgesehen sind, mittels derer bei einer Inkonsistenz zwischen dem durch die Zugabevorrichtung aufgenommenen Flüssigkeitsvolumen und dem gemessenen Druck eine Information des Anwenders erfolgt.

18. Dialysemaschine nach einem der Ansprüche 11 bis 17, **dadurch gekennzeichnet, dass** Eingabemittel vorgesehen sind, mittels derer die patientenindividuelle Konzentrationen des zuzugebenden Mittels eingebbar ist.

19. Dialysemaschine nach einem der Ansprüche 11 bis 18, **dadurch gekennzeichnet, dass** die Eingabemittel eine Leseeinrichtung umfassen, mittels derer eine patientenindivuduelle Medikation von einer Karte oder einem sonstigen Speichermittel auslesbar ist.

20. Dialysemaschine nach einem der Ansprüche 11 bis 19, **dadurch gekennzeichnet, dass** die Zugabevorrichtung mit einer von mehreren standardisierten Mengen und/oder Konzentrationen des Konzentrats gefüllt ist und dass die Dialysemaschine Eingabemittel aufweist, in die eingebbar ist, um welches der standardisierten Konzentrate es sich handelt.

**21.** Dialysemaschine nach einem der Ansprüche 11 bis 20, **dadurch gekennzeichnet, dass** der extrakorporale Kreislauf (20) ein Kassettensystem umfaßt.

**Claims**

**1.** A method for the filling of a metering apparatus of a therapy device having an extracorporeal circuit (20) with which the metering apparatus is in communication such that an agent can be introduced into the extracorporeal circuit (20) during the operation of the therapy device by means of the metering device, with the method comprising the step of the dilution of a concentrate located in the metering apparatus with a diluting agent, wherein the dilution of the concentrate located in the metering apparatus takes place in that the metering apparatus takes up the diluting agent from the extracorporeal circuit (20) in the condition connected to the extracorporeal circuit (20), **characterized in that** the method comprises the step of flushing the extracorporeal circuit; and **in that** the extracorporeal circuit (20) comprises a blood tubing set or consists of it; and **in that** the taking up of the diluting agent by the metering apparatus takes place before the start of the treatment and during the flushing phase of the extracorporeal circuit (20) and preferably briefly before the end of the flushing phase of the extracorporeal circuit (20.

**2.** A method in accordance with claim 1, wherein the metering apparatus is filled with a standardized amount and/or concentration of the concentrate.

**3.** A method in accordance with either of claims 1 or 2, wherein the metering apparatus is a syringe pump (30) with a syringe (10) inserted therein; and wherein the taking up of the diluting agent takes place by drawing up the syringe (10).

**4.** A method in accordance with claim 1, wherein the flushing of the extracorporeal circuit (20) takes place with saline solution or with dialysate preferably manufactured online; and wherein the diluting agent is accordingly saline solution or dialysate.

**5.** A method in accordance with one of the preceding claims, wherein the concentrate is an anticoagulant.

**6.** A method in accordance with claim 5, wherein the anticoagulant is heparin.

**7.** A method in accordance with one of the preceding claims, wherein a pressure sensor (50) in communication with the extracorporeal circuit (20) is provided; wherein a monitoring of the pressure measured by means of the pressure sensor (50) takes place during the take-up of the diluting agent by the metering apparatus; and wherein, in the event of inconsistency between the fluid volume taken up by the metering apparatus and the pressure measured, information is given to the user.

**8.** A method in accordance with one of the preceding claims, wherein input means are provided by means of which the concentration of the agent to be metered can be input individually for the patient; and wherein a control unit is provided which determines the required volume of the diluting agent in an automated manner on the basis of the data received from the input means and controls the metering apparatus such that it takes up the determined volume of the diluting agent.

**9.** A method in accordance with claim 8, wherein the input means comprise a reading device by means of which a medication individual to the patient can be read out from a card or from another memory means.

**10.** A method in accordance with one of the preceding claims, wherein the metering apparatus is filled with one of a plurality of standardized amounts and/or concentrations of the concentrate; and wherein the therapy device has input means into which it can be input which of the standardized concentrates it is.

**11.** A dialysis machine, suitable for the taking up of an extracorporeal circuit (20) comprising a metering apparatus for concentrate having a diluting agent, wherein the dialysis machine has a control unit and input means, **characterized in that** the control unit is made such that it determines the required volume of the diluting agent in an automated manner on the basis of the data received from the input means and controls the metering apparatus such that it takes up the determined volume of the diluting agent from the extracorporeal circuit (20), wherein the control unit is made such that the taking up of the diluting agent by the metering apparatus takes place during the flushing phase of the extracorporeal circuit (20) and preferably briefly before the end of the flushing phase of the extracorporeal circuit (20).

**12.** A dialysis machine in accordance with claim 11, wherein the metering apparatus is filled with a standardized amount and concentration of the concentrate.

**13.** A dialysis machine in accordance with either of claims 11 or 12, wherein the metering apparatus is a syringe pump (30) having a syringe (10) inserted therein, with the metering apparatus being arranged such that the taking up of the diluting agent from the extracorporeal circuit (20) takes place by drawing up the syringe (10).

**14.** A dialysis machine in accordance with claim 11, wherein a saline solution or a dialysate preferably manufactured online is provided as the flushing means for the flushing of the extracorporeal circuit (20); and wherein the diluting agent is accordingly saline solution or dialysate.

**15.** A dialysis machine in accordance with any of the claims 11 to 14, wherein the concentrate is an anticoagulant.

**16.** A dialysis machine in accordance with claim 15, wherein the anticoagulant is heparin.

**17.** A dialysis machine in accordance with one of the claims 11 to 16, wherein a pressure sensor (50) in communication with the extracorporeal circuit (20) is provided; wherein monitoring means are provided by means of which a monitoring of the pressure measured by means of the pressure sensor (50) takes place during the taking up of the diluting agent by the metering apparatus; and wherein information means are provided by means of which, in the event of inconsistency between the fluid volume taken up by the metering apparatus and the pressure measured, information is given to the user.

**18.** A dialysis machine in accordance with one of the claims 11 to 17, wherein input means are provided by means of which the concentrations of the agent to be metered individual to the patient can be input.

**19.** A dialysis machine in accordance with one of the claims 11 to 18, wherein the input means comprise a reading device by means of which a medication individual to the patient can be read off from a card or from another memory means.

**20.** A dialysis machine in accordance with one of the claims 11 to 19, wherein the metering apparatus is filled with one of a plurality of standardized amounts and/or concentrations of the concentrate; and wherein the dialysis machine has input means into which it can be input which of the of the standardized concentrates it is.

**21.** A dialysis machine in accordance with one of the claims 11 to 20, wherein the extracorporeal circuit (20) comprises a cassette system.

**Revendications**

**1.** Procédé pour remplir un dispositif d'alimentation d'un appareil thérapeutique, qui comporte un circuit extracorporel (20), avec lequel le dispositif d'alimentation est en liaison de telle manière qu'un produit peut être introduit dans le circuit extracorporel (20) au moyen du dispositif d'alimentation pendant le fonctionnement de l'appareil thérapeutique, le procédé comprenant l'étape consistant à diluer un concentré se trouvant dans le dispositif d'alimentation avec un diluant, la dilution du concentré se trouvant dans le dispositif d'alimentation se faisant par le fait que le dispositif d'alimentation, dans l'état raccordé au circuit extracorporel (20), reçoit le diluant en provenance du circuit extracorporel (20), **caractérisé en ce que** le procédé comprend l'étape consistant à rincer le circuit extracorporel et **en ce que** le circuit extracorporel (20) comprend un ensemble de tubes sanguins ou est constitué de celui-ci et **en ce que** la réception du diluant par le dispositif d'alimentation se fait avant le début du traitement et pendant la phase de rinçage du circuit extracorporel (20) et de préférence peu avant la fin de la phase de rinçage du circuit extracorporel (20).

**2.** Procédé selon la revendication 1 **caractérisé en ce que** le dispositif d'alimentation est rempli avec une quantité et/ou une concentration standard du concentré.

**3.** Procédé selon la revendication 1 ou 2, **caractérisé en ce que** le dispositif d'alimentation est un pousse-seringue (30) comprenant une seringue (10) placée dans celui-ci et **en ce que** la réception du diluant se fait par aspiration dans la seringue (10).

**4.** Procédé selon la revendication 1, **caractérisé en ce que** le rinçage du circuit extracorporel (20) se fait avec de la solution physiologique ou avec du dialysat produit de préférence en ligne et **en ce que** le diluant est en conséquence de la solution physiologique ou du dialysat.

**5.** Procédé selon l'une des revendications précédentes, **caractérisé en ce que** le concentré est un anticoagulant.

**6.** Procédé selon la revendication 5, **caractérisé en ce que** l'anticoagulant est de l'héparine.

**7.** Procédé selon l'une des revendications précédentes, **caractérisé en ce qu'**un capteur de pression (50) qui est en liaison avec le circuit extracorporel (20) est prévu, **en ce qu'**une surveillance de la pression mesurée au moyen du capteur de pression (50) est effectuée pendant la réception du diluant par le dispositif d'alimentation, et **en ce qu'**en cas d'incohérence entre le volume de liquide reçu par le dispositif d'alimentation et la pression mesurée, une information de l'utilisateur a lieu.

**8.** Procédé selon l'une des revendications précédentes, **caractérisé en ce que** des moyens de saisie sont prévus, au moyen desquels la concentration du produit à alimenter personnalisée pour le patient peut être saisie et **en ce qu'**une unité de commande est prévue, qui détermine automatiquement le volume requis du diluant sur la base des données obtenues par les moyens de saisie et qui commande le dispositif d'alimentation de telle manière que celui-ci reçoit le volume déterminé du diluant.

**9.** Procédé selon la revendication 8, **caractérisé en ce que** les moyens de saisie comprennent un dispositif de lecture, au moyen duquel une médication personnalisée pour le patient peut être lue à partir d'une carte ou d'un autre support de mémoire.

**10.** Procédé selon l'une des revendications précédentes, **caractérisé en ce que** le dispositif d'alimentation est rempli avec une parmi plusieurs quantités et/ou concentrations standard du concentré et **en ce que** l'appareil thérapeutique comporte des moyens de saisie, dans lesquels il est possible de saisir duquel des concentrés standard il s'agit.

**11.** Machine de dialyse, adaptée pour la réception d'un circuit extracorporel (20), comprenant un dispositif d'alimentation pour concentré avec un diluant, la machine de dialyse comportant une unité de commande ainsi que des moyens de saisie, **caractérisée en ce que** l'unité de commande est conçue de telle manière qu'elle détermine automatiquement le volume du diluant requis sur la base des données obtenues par les moyens de saisie et qu'elle commande le dispositif d'alimentation de telle manière que celui-ci reçoit le volume déterminé du diluant en provenance du circuit extracorporel (20), l'unité de commande étant conçue de telle manière que la réception du diluant par le dispositif d'alimentation se fait pendant la phase de rinçage du circuit extracorporel (20) et de préférence peu avant la fin de la phase de rinçage du circuit extracorporel (20).

**12.** Machine de dialyse selon la revendication 11 **caractérisée en ce que** le dispositif d'alimentation est rempli avec une quantité et une concentration standard du concentré.

**13.** Machine de dialyse selon la revendication 11 ou 12, **caractérisée en ce que** le dispositif d'alimentation est un pousse-seringue (30) comprenant une seringue (10) placée dans celui-ci, le dispositif d'alimentation étant disposé de telle manière que la réception du diluant en provenance du circuit extracorporel (20) se fait par aspiration dans la seringue (10).

**14.** Machine de dialyse selon la revendication 11, **caractérisée en ce qu'**une solution physiologique ou du dialysat produit de préférence en ligne est prévu(e) en tant qu'agent de rinçage pour le rinçage du circuit extracorporel (20) et **en ce que** le diluant est en conséquence de la solution physiologique ou du dialysat.

**15.** Machine de dialyse selon l'une des revendications 11 à 14, **caractérisée en ce que** le concentré est un anticoagulant.

**16.** Machine de dialyse selon la revendication 15, **caractérisée en ce que** l'anticoagulant est de l'héparine.

**17.** Machine de dialyse selon l'une des revendications 11 à 16, **caractérisée en ce qu'**un capteur de pression (50) qui est en liaison avec le circuit extracorporel (20) est prévu, **en ce que** des moyens de surveillance sont prévus, au moyen desquels une surveillance de la pression mesurée au moyen du capteur de pression (50) est effectuée pendant la réception du diluant par le dispositif d'alimentation, et **en ce que** des moyens d'information sont prévus, au moyen desquels une information de l'utilisateur a lieu en cas d'incohérence entre le volume de liquide reçu par le dispositif d'alimentation et la pression mesurée.

**18.** Machine de dialyse selon l'une des revendications 11 à 17, **caractérisée en ce que** des moyens de saisie sont prévus, au moyen desquels les concentrations du fluide à alimenter personnalisées pour le patient peuvent être saisies.

**19.** Machine de dialyse selon l'une des revendications 11 à 18, **caractérisée en ce que** les moyens de saisie comprennent un dispositif de lecture, au moyen duquel une médication personnalisée pour le patient peut être lue à partir d'une carte ou d'un autre support de mémoire.

**20.** Machine de dialyse selon l'une des revendications 11 à 19, **caractérisée en ce que** le dispositif d'alimentation est rempli avec une parmi plusieurs quantités et/ou concentrations standard du concentré et en ce que la machine de dialyse comporte des moyens de saisie, dans lesquels il est possible de saisir duquel des concentrés standard il s'agit.

**21.** Machine de dialyse selon l'une des revendications 11 à 20, **caractérisée en ce que** le circuit extracorporel (20) comprend un système de cassette.

# Figur 1

# Figur 2

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- US 5015226 A
- US 20050234382 A1
- US 5116316 A